# EUROPÄISCHE PATENTANMELDUNG

(11) **EP 4 792 864 A1**
(43) Veröffentlichungstag der Anmeldung: **19.08.2026**
(21) Anmeldenummer: 26157898.3
(22) Anmeldetag: 11.02.2026
(51) Int. Cl.: A61M 16/08

(54) **FLEXIBLER BEATMUNGSSCHLAUCH**

(30) Priorität: 12.02.2025 DE 102025105230
(71) Anmelder: Löwenstein Medical Technology S.A., 2557 Luxembourg (LU)
(72) Erfinder: Schulze, Willy, 90552 Röthenbach an der Pegnitz (DE)
(74) Vertreter: Löwenstein Medical IP

(57) **Zusammenfassung**

Die Erfindung betrifft einen Beatmungsschlauch (1) mit einer durchgehenden Wand (2), die ein inneres Lumen begrenzt, wobei das Lumen zur Leitung von Atemgas ausgebildet ist, wobei eine Helix (3) die Wand (2), auf der dem Lumen abgewandten Seite spiralförmig umgibt, wobei die Wand (2) aus einem ersten Material hergestellt wird und wobei die Helix (3) aus einem zweiten Material hergestellt wird, wobei die Helix (3) mit der Wand (2) verbunden ist. Der Schlauch (1) zeichnet sich dadurch aus, dass er formstabil und trotzdem flexibel ist.

## Beschreibung

Die vorliegende Erfindung betrifft einen flexiblen Beatmungsschlauch für die medizinische Beatmung, insbesondere zur Anwendung in CPAP-, BiPAP- oder invasiven Beatmungssystemen. Der Beatmungsschlauch weist eine durchgehende elastische Membran auf, welche ein inneres Lumen zur Leitung von Atemgas begrenzt, sowie eine die Membran auf der dem Lumen abgewandten Seite spiralförmig umgebende Helix.

### Stand der Technik

Herkömmliche Beatmungsschläuche besitzen in der Regel ein ausgeprägtes Rückstellvermögen. Wird ein solcher Schlauch gebogen oder ausgelenkt, so wirkt eine Rückstellkraft, die den Schlauch in seine Ausgangsform zurückzuführen versucht. Diese Rückstellkraft kann sich in Form einer Zugbelastung auf die Beatmungsschnittstelle, insbesondere auf eine Atemmaske oder einen Tubus, übertragen.

Eine derartige Zugbelastung wird von Patienten häufig als unangenehm empfunden und kann den Schlaf oder die Therapietoleranz beeinträchtigen. Darüber hinaus kann eine erhöhte Zugbelastung zu Leckagen zwischen Maske und Gesicht führen, wodurch der Therapieerfolg gefährdet wird.

Aus dem Stand der Technik sind flexible Beatmungsschläuche bekannt, die jedoch entweder eine vergleichsweise hohe Steifigkeit oder eine hohe Dehnbarkeit aufweisen. So offenbart beispielsweise die DE 10 2024 110 733 A1 einen Schlauch mit einer Federrate von mindestens 25 N/m. Die EP 2 438 953 B1 beschreibt einen Schlauch, der auf 40 % bis 400 % seiner ursprünglichen Länge dehnbar ist. Solche Schläuche zeigen jedoch entweder eine hohe Rückstellkraft oder eine unzureichende Formstabilität, insbesondere unter rein gravitationsbedingter Belastung.

### Aufgabe der Erfindung

Vor diesem Hintergrund liegt der vorliegenden Erfindung die Aufgabe zugrunde, einen Beatmungsschlauch bereitzustellen, der eine geringe Rückstellkraft aufweist und dadurch eine reduzierte Zugbelastung auf die Beatmungsschnittstelle ausübt, dabei jedoch eine ausreichende Form- und Knickstabilität beibehält.

### Lösung der Aufgabe

Diese Aufgabe wird durch einen Beatmungsschlauch mit den Merkmalen des Anspruchs 1 gelöst. Vorteilhafte Weiterbildungen und Ausgestaltungen sind Gegenstand der Unteransprüche.

Der erfindungsgemäße Beatmungsschlauch weist eine durchgehende, elastische Membran auf, die ein inneres Lumen zur Leitung von Atemgas begrenzt. Eine Helix umgibt die Membran auf der dem Lumen abgewandten Seite spiralförmig. Die Membran besteht aus einem ersten polymeren Material, während die Helix aus einem zweiten, hiervon verschiedenen polymeren Material besteht. Die Helix ist formschlüssig und/oder stoffschlüssig mit der Membran verbunden.

Erfindungsgemäß beträgt die Rückstellkraft des Beatmungsschlauchs, gemessen gemäß dem in der Beschreibung erläuterten Prüfverfahren, höchstens 0,6 N, bevorzugt höchstens 0,4 N, besonders bevorzugt höchstens 0,2 N.

Weiterhin zeichnet sich der Beatmungsschlauch dadurch aus, dass bei einer Biegeprüfung unter ausschließlicher Einwirkung des Eigengewichts des Schlauches die horizontale Auslenkung des Schlauches mit zunehmender vertikaler Auslenkung zunächst nichtlinear zunimmt und anschließend einen im Wesentlichen konstanten Wert erreicht.

Dieses Verhalten wird im Rahmen der vorliegenden Anmeldung als besonders günstiges Drapierverhalten bewertet, da der Schlauch bereits bei geringer vertikaler Auslenkung seine maximale Biegeverformbarkeit erreicht und bei weiterer Belastung keine signifikante zusätzliche horizontale Auslenkung zeigt.

### Drapierverhalten und Rückstellkraft

Bei bevorzugten Ausführungsformen weist der Schlauch ab einer vertikalen Auslenkung von höchstens 150 mm bei weiterer Erhöhung der vertikalen Auslenkung eine Änderung der horizontalen Auslenkung von weniger als 10 % auf.

Die horizontale Auslenkung beträgt bei einer vertikalen Auslenkung von 100 mm bevorzugt höchstens 100 mm.

Das beschriebene Drapierverhalten kann mathematisch näherungsweise durch eine asymptotische Funktion beschrieben werden. Diese mathematische Beschreibung dient insbesondere der Erläuterung des Kurvenverlaufs.

### Temperaturverhalten

Bei bevorzugten Ausführungsformen unterscheidet sich die Rückstellkraft des Schlauchs bei einer Temperatur von 20 °C und bei einer Temperatur von 40 °C um weniger als 20 %. Dies ist insbesondere für Beatmungssysteme mit temperiertem und befeuchtetem Atemgas von Vorteil.

### Materialien und Geometrie

Die Membran besteht bevorzugt aus einem thermoplastischen Elastomer (TPE), TPE-S oder Silikon.

Die Wanddicke der Membran liegt bevorzugt im Bereich von 0,1 mm bis 0,3 mm.

Die Helix besteht bevorzugt aus TPE, TPE-S oder Silikon und weist einen Durchmesser von 0,4 mm bis 2,5 mm auf.

Die Helix verleiht dem Schlauch eine Knickstabilität, sodass bei einer Biegung mit einem Radius von 20 mm kein Zusammenfallen des Lumens auftritt.

Membran und Helix können bevorzugt im Mehrkomponenten-Spritzgussverfahren oder durch Umspritzen hergestellt sein.

Die Drapierbarkeit des Schlauches kann als asymptotische Funktion wie
∘ $f \left(x\right) = a - b ⋅ {e}^{- c ⋅ x}$
beschrieben werden.

Die horizontale Ausdehnung (Horizontal Distance Out) des Schlauches bei vertikaler Belastung mit dem Eigengewicht des Schlauches (Vertikal Distance down) ist annähernd gleichgroß (bis zu +15% größer) wie die (Schlauch-Länge der) angelegte(n) vertikale Belastung (Vertikal Distance down), oder kleiner.

Die horizontale Ausdehnung des Schlauches bei vertikaler Belastung mit dem Eigengewicht des Schlauches ist bevorzugt, zumindest für geringere Belastungen unter 100 mm, kleiner als die Länge der angelegten vertikale Belastung.

Bevorzugt verhält sich der Schlauch hinsichtlich Drapierbarkeit und Rückstellkraft bei 20°C sehr ähnlich wie bei 40°C.

Der erfindungsgemäße Beatmungsschlauch weist eine durchgehende Membran auf, die hergestellt ist aus TPE oder TPE-S oder Silikon.

Der erfindungsgemäße Beatmungsschlauch weist eine durchgehende Membran auf, deren Wanddicke 0,1 mm bis 2 mm, bevorzugt 0,15 mm ist.

Bevorzugt ist die Helix hergestellt aus TPE oder Polyolefinen und der Durchmesser der Helix liegt im Bereich 0,4 bis 2,5 mm, bevorzugt 2 mm.

Das Rückstellvermögen ist insbesondere in einem Temperaturbereich von 20° bis 40°C wenig veränderlich. Was wiederum ein deutlicher Vorteil bei Atemtherapien, bei welchem das Atemgas in temperierter Form verabreicht wird, bietet. Üblicherweise werden in der Praxis, je nach Therapieform, Atemgastemperaturen von bis zu 40°C und 100% Luftfeuchtigkeit erreicht.

Die oben beschriebenen Vorteile werden hauptsächlich über die materialspezifischen Eigenschaften, insbesondere von der sogenannten Membran (welche das Innenlumen umschließt) gebildet.

Die Flexibilität bzw. das Rückstellvermögen unterscheidet sich allerdings entscheidend von allen bekannten, sich auf dem Markt befindlichen, geometrisch vergleichbaren Schläuchen.
**Figur 1** zeigt einen Beatmungsschlauch mit einer durchgehenden Membran, die ein inneres Lumen begrenzt, sowie einer Helix.
**Figur 2** zeigt eine Messvorrichtung zur Bestimmung der Rückstellkraft des Beatmungsschlauchs.
**Figuren 3** **und** **4** zeigen Messergebnisse zur Rückstellkraft und zur Drapierbarkeit verschiedener Schlauchtypen.
**Figuren 5a bis 5d** veranschaulichen das Messverfahren zur Bestimmung der horizontalen Auslenkung eines Schlauchs bei zunehmender vertikaler Auslenkung unter Einwirkung des Eigengewichts.

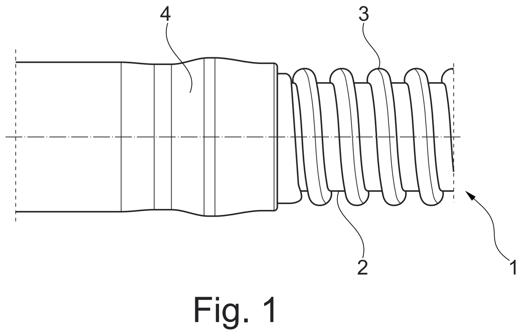

Fig. 1 zeigt einen Beatmungsschlauch (1) mit einer durchgehenden Membran (2) die ein inneres Lumen begrenzt. Das Lumen ist zur Leitung von Atemgas ausgebildet. Eine Helix (3) umgibt die Membran (2) auf der dem Lumen abgewandten Seite spiralförmig.

Die Membran (2) aus einem ersten Material hergestellt ist und die Helix (3) aus
einem zweiten Material hergestellt. Der Innendurchmesser ist 15 - 22 mm. Die Rückstellkraft des Schlauches ist bevorzugt unter 0,6 N und besonders bevorzugt unter 0,4 N und ganz besonders bevorzugt bei 0,2 N.

Der Schlauch kann an einem Ende eine Muffe aufweisen, die dem Anschluss an ein Beatmungsgerät dient. Das andere nicht gezeigte Ende des Schlauches kann einen Adapter aufweisen, der der Ankopplung einer Maske dient.

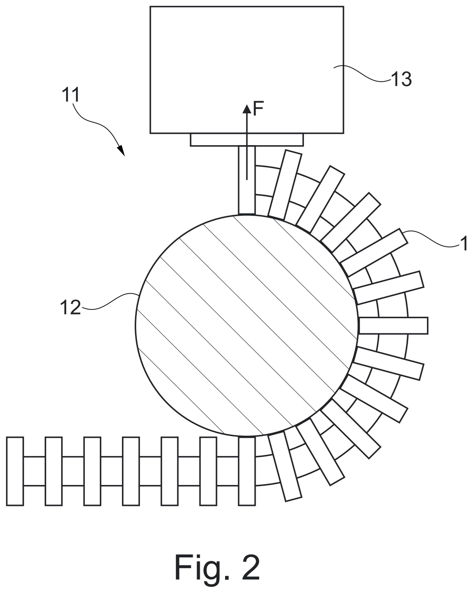

Fig. 2 zeigt einen Beatmungsschlauch (1) nach Fig. 1, der in eine Messvorrichtung (11) zur Bestimmung der Rückstellkraft in Newton (N) eingelegt ist.

Die Rückstellkraft des Schlauches wurde folgendermaßen ermittelt:
Der Schlauch (1) wird mit einer entsprechenden Länge, horizontal an einen Rundkörper (12) (eine kreisrunde oder ovale Vorrichtung) mit dem Durchmesser von beispielsweise 90mm angelegt. Hierbei muss der der Schlauch, etwa an der Hälfte des Umfangs des Rundkörpers anliegen. Der Teil des Schlauches, der sich nicht an dem Rundkörper befindet, liegt dabei gerade und unbelastet auf einer ebenen Fläche. Genau mittig über dem Rundkörper ist eine Kraftmessdose (5) angebracht. Das obere Ende des Schlauches drückt dann, durch das Rückstellvermögen des Schlauches gegen die Kraftmessdose (5). Die hierbei auftretende Kraft F ist die Rückstellkraft des Schlauches.

Eine geringe Rückstellkraft bei einem Schlauch bedeutet, dass der Schlauch weniger Widerstand leistet, wenn er gebogen oder verformt wird, und sich nur mit geringer Kraft in seine ursprüngliche Form zurückzieht.

Die Bedeutung einer geringen Rückstellkraft liegt in der höheren Flexibilität. Der Schlauch ist weicher und lässt sich leichter biegen, ohne starken Widerstand zu erzeugen. Das kann besonders vorteilhaft sein, wenn Bewegungsfreiheit gefragt ist.

Es wurde die Rückstellkraft und Drapierbarkeit verschiedener Schläuche vom Durchmesser Ø15mm ermittelt.

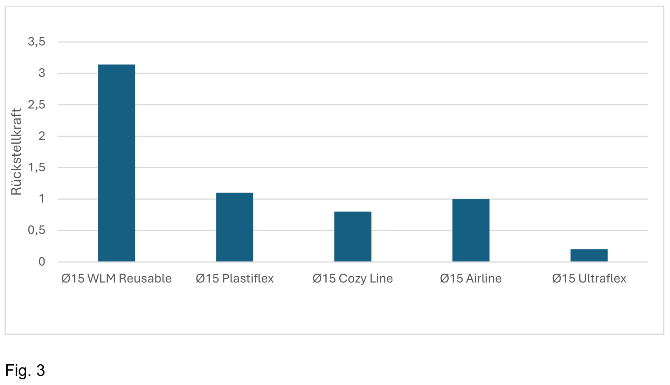

Fig. 3 zeigt für verschiedene Schläuchen die jeweilige Rückstellkraft in Newton (N). Analyse der Werte:
- WLM Reusable hat mit 3,14 N die höchste Rückstellkraft.
- UtraFlex (UF) hat mit 0,2 N die geringste Rückstellkraft.
- Die anderen Schläuche liegen dazwischen:
   ∘ Plastiflex: 1,1 N
   ∘ Cozy Line: 0,8 N
   o Airline: 1,0 N

Das Diagramm visualisiert die Unterschiede deutlich, wobei der Ø15 WLM Reusable-Schlauch am höchsten und der Ø15 UF-Schlauch am niedrigsten dargestellt ist.

Die Rückstellkraft eines Schlauches hängt von mehreren Faktoren ab, darunter das Material, der Durchmesser, die Wandstärke und die Deformation (z. B. Biegung oder Dehnung), der der Schlauch ausgesetzt wird.

Um die Rückstellkraft F eines Schlauchs zu berechnen, kann man eine vereinfachte Annäherung mit Hilfe des Hooke'schen Gesetzes verwenden, wenn die Deformation elastisch ist: $F = k ⋅ ΔxF = k \ cdot \ Delta xF = k ⋅ Δx$

Dabei ist:
- F die Rückstellkraft (in Newton),
- k die Federkonstante oder Steifigkeit des Schlauchs (in N/m),
- Δx\Delta xΔx die Deformation (Verlängerung oder Verkürzung) des Schlauchs (in Metern).

Die Federkonstante k hängt ab von:
- dem Materialmodul (E-Modul) des Kunststoffs,
- der Geometrie des Schlauchs (Durchmesser, Wandstärke).

Für eine genauere Berechnung sind spezifische Materialdaten und die genaue Geometrie des Schlauchs relevant. In der Praxis kann die Rückstellkraft auch durch experimentelle Messung bestimmt werden, indem man den Schlauch um eine bekannte Länge verformt und die erforderliche Kraft misst.

Weniger Zugkräfte auf den Patienten: In der medizinischen Anwendung, z. B. bei Beatmungsschläuchen, bedeutet dies, dass der Schlauch weniger an der Maske oder dem Tubus zieht, was den Komfort des Patienten verbessert. Einfache Handhabung: Schläuche mit niedriger Rückstellkraft sind oft angenehmer zu positionieren und passen sich besser an verschiedene Liege- oder Sitzpositionen an. Stabilitätsverlust möglich: Eine sehr geringe Rückstellkraft kann jedoch dazu führen, dass der Schlauch leicht einknickt oder seine Form nicht gut hält, was den Luftfluss beeinträchtigen könnte. Zusammengefasst: Ein Schlauch mit geringer Rückstellkraft ist flexibler und komfortabler, aber möglicherweise weniger stabil. Vorliegend wird die Stabilität auch über die Helix mitbestimmt.

Bedeutung einer geringen Rückstellkraft:
1. Höhere Flexibilität: Der Schlauch ist weicher und lässt sich leichter biegen, ohne starken Widerstand zu erzeugen. Das kann besonders vorteilhaft sein, wenn Bewegungsfreiheit gefragt ist.
2. Weniger Zugkräfte auf den Patienten: In der medizinischen Anwendung, z. B. bei Beatmungsschläuchen, bedeutet dies, dass der Schlauch weniger an der Maske oder dem Tubus zieht, was den Komfort des Patienten verbessert.
3. Einfache Handhabung: Schläuche mit niedriger Rückstellkraft sind oft angenehmer zu positionieren und passen sich besser an verschiedene Liege- oder Sitzpositionen an.
4. Stabilitätsverlust möglich: Eine sehr geringe Rückstellkraft kann jedoch dazu führen, dass der Schlauch leicht einknickt oder seine Form nicht gut hält, was den Luftfluss beeinträchtigen könnte.

Die Rückstellkraft eines Schlauchs hat einen direkten Einfluss auf seine Flexibilität, Stabilität und Handhabung.
1. Hohe Rückstellkraft (z. B. WLM Reusable - 3,14 N)
   - Geeignet für Anwendungen mit hoher mechanischer Belastung
   Nachteile:
   - Weniger flexibel, schwerer zu positionieren
   - Kann stärkeren Zug auf die Beatmungsschnittstelle (z. B. Maske oder Tubus) ausüben
2. Mittlere Rückstellkraft (z. B. Plastiflex - 1,1 N, Airline - 1,0 N, Cozy Line - 0,8 N)
   - Guter Kompromiss zwischen Flexibilität und Stabilität
   Nachteile:
   - Kann in manchen Situationen noch leicht knicken
3. Sehr geringe Rückstellkraft (z. B. UF - 0,2 N)
   Vorteile:
   - Sehr flexibel, passt sich leicht an Bewegungen an
   - Minimaler Zug auf den Patienten, hoher Komfort
   - Ideal für mobile oder empfindliche Patienten

Der UF-Schlauch ist der flexibelste. Er eignet sich besonders für Anwendungen, bei denen Bewegungsfreiheit und Tragekomfort entscheidend sind.

Die Drapierbarkeit eines Schlauches bezieht sich auf die Fähigkeit des Schlauchs, sich an eine bestimmte Form oder Oberfläche anzupassen, ohne starke Widerstandskräfte auszuüben. Die Drapierbarkeit eines Schlauches wird mittels einer Biegeprüfung unter ausschließlicher Einwirkung des Eigengewichts des Schlauches bestimmt. Die Drapierbarkeit eines Schlauches beschreibt also, wie flexibel oder steif der Schlauch ist und wie gut er sich unter seinem eigenen Gewicht oder durch äußere Einwirkung biegen lässt, ohne in seine ursprüngliche Form zurückzukehren.

Faktoren, die die Drapierbarkeit beeinflussen:
1. Materialeigenschaften:
   ∘ Elastizitätsmodul (E-Modul): Ein niedriges E-Modul führt zu höherer Flexibilität und besserer Drapierbarkeit.
   ∘ Dichte: Schwerere Materialien können unter ihrem eigenen Gewicht besser drapieren.
2. Wandstärke:
   ∘ Dünnwandige Schläuche sind drapierfähiger als dickwandige, da sie leichter biegbar sind.
3. Durchmesser:
   ∘ Schläuche mit kleinem Durchmesser sind in der Regel flexibler und drapierfähiger als solche mit großem Durchmesser.
4. Temperatur:
   ∘ Viele Kunststoffe werden bei höheren Temperaturen weicher, was die Drapierbarkeit verbessert.
5. Weichmacheranteil:
   ∘ Kunststoffe mit einem höheren Weichmacheranteil sind flexibler und weisen eine bessere Drapierbarkeit auf.

### Anwendungsbeispiel:

Ein Schlauch mit hoher Drapierbarkeit eignet sich gut für Anwendungen, bei denen der Schlauch sich um Ecken oder unregelmäßige Formen legen muss, wie z. B. in der Medizintechnik oder bei der Installation in engen Räumen.

Die Quantitative Ermittlung der Flexibilität / des Rückstellvermögens erfolgt über einen den Drapierbarkeitstest. Die Drapierbarkeit eines Schlauches wird mittels einer Biegeprüfung unter ausschließlicher Einwirkung des Eigengewichts des Schlauches bestimmt, wobei die horizontale Auslenkung des Schlauches und/oder die vertikale Auslenkung bestimmt werden.

Die horizontale Ausdehnung (Sample Horizontal Distance Out in mm) der Schläuche wird in Abhängigkeit von der vertikalen Belastung durch das Eigengewicht des jeweiligen Schlauches bestimmt.

**Tab. 1 Drapierbarkeit**

| **UF Ø15** | Vertical Distance Down in mm - Sample Horizontal Distance Out in mm | | | | | | | | | | | |
|---|---|---|---|---|---|---|---|---|---|---|---|---|
| *Schlauch Markierung* mm | **25** | **50** | **75** | **100** | **125** | **150** | **175** | **200** | **225** | **250** | **275** | **300** |
| *300* | 25 | 30 | 38 | 43 | 46 | 48 | 48 | 48 | 48 | 48 | 48 | 48 |
| *400* | 18 | 26 | 32 | 36 | 38 | 40 | 40 | 40 | 40 | 40 | 40 | 40 |
| *600* | 18 | 24 | 27 | 30 | 32 | 32 | 32 | 32 | 32 | 32 | 32 | 32 |

| **WLM Standard Ø15** | Vertical Distance Down in mm - Sample Horizontal Distance Out in mm | | | | | | | | | | | |
|---|---|---|---|---|---|---|---|---|---|---|---|---|
| *Schlauch Markierung* mm | **25** | **50** | **75** | **100** | **125** | **150** | **175** | **200** | **225** | **250** | **275** | **300** |
| *300* | 156 | 250 | | | | | | | | | | |
| *400* | 85 | 137 | 182 | 220 | 256 | 295 | | | | | | |
| *600* | 36 | 60 | 80 | 95 | 110 | 125 | 136 | 145 | 155 | 165 | 172 | 180 |

| **WLM Reuse Ø15** | Vertical Distance Down in mm - Sample Horizontal Distance Out in mm | | | | | | | | | | | |
|---|---|---|---|---|---|---|---|---|---|---|---|---|
| *Schlauch Markierung* mm | **25** | **50** | **75** | **100** | **125** | **150** | **175** | **200** | **225** | **250** | **275** | **300** |
| *300* | 70 | 95 | 120 | 160 | 190 | 220 | | | | | | |
| *400* | 70 | 85 | 100 | 110 | 120 | 140 | 145 | 155 | 164 | 175 | 185 | 195 |
| *600* | 55 | 80 | 95 | 110 | 120 | 130 | 140 | 145 | 145 | 145 | 145 | 145 |

| **Plastiflex 015** | Vertical Distance Down in mm - Sample Horizontal Distance Out in mm | | | | | | | | | | | |
|---|---|---|---|---|---|---|---|---|---|---|---|---|
| *Schlauch Markierung* mm | **25** | **50** | **75** | **100** | **125** | **150** | **175** | **200** | **225** | **250** | **275** | **300** |
| *300* | 40 | 55 | 65 | 75 | 85 | 95 | 110 | | | | | |
| *400* | 92 | 125 | 155 | 187 | 210 | 230 | 88 | 90 | 95 | 100 | 105 | |
| *600* | 55 | 72 | 90 | 102 | 115 | 120 | 125 | 132 | 132 | 132 | 132 | 132 |

| **Cozy Line Ø15** | Vertical Distance Down in mm - Sample Horizontal Distance Out in mm | | | | | | | | | | | |
|---|---|---|---|---|---|---|---|---|---|---|---|---|
| *Schlauch Markierung* mm | **25** | **50** | **75** | **100** | **125** | **150** | **175** | **200** | **225** | **250** | **275** | **300** |
| *300* | 50 | 73 | 95 | 118 | 143 | 37 | 37 | 37 | 37 | 37 | 37 | 37 |
| *400* | 45 | 63 | 78 | 88 | 95 | 100 | 60 | 65 | 70 | 85 | 90 | 100 |
| *600* | 30 | 38 | 43 | 48 | 50 | 50 | | | | | | |

Das Verhältnis aus "Horizontaler Verformung" zu "vertikaler Verformung" gibt ein Maß für die Flexibilität an.

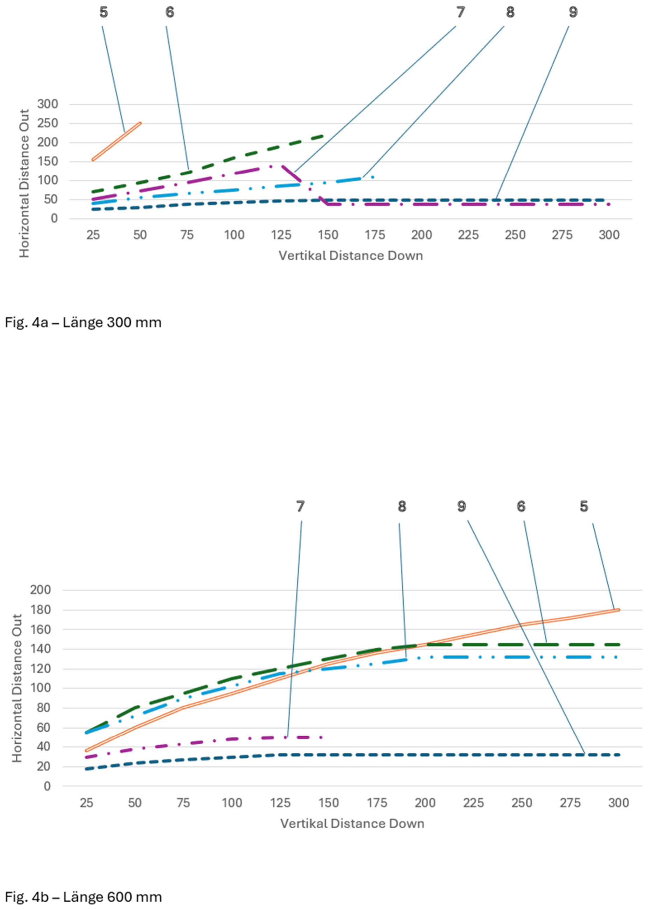

Die Messwerte der Tabelle 1 sind aufgetragen in den Figuren 4 a und 4b.

### Fig 4 Drapierbarkeit

Die Figuren zeigen Messergebnisse von Schläuchen. Hierbei wird die horizontale Ausdehnung (Sample Horizontal Distance Out in mm) der Schläuche in Abhängigkeit von der vertikalen Belastung (Vertical Distance Down in mm) für unterschiedliche Schlauchlängen (300 mm, 600 mm) erfasst.

Die Messwerte stammen von fünf verschiedenen Schlauchtypen mit dem Durchmesser Ø15 mm:
(9) UF
WLM Standard (5)
WLM Reuse (6)
Plastiflex (8)
Cozy Line (7)

### Analyse der Messergebnisse

1. UF (9)
   - Zeigt die geringste horizontale Ausdehnung bei Belastung. Die horizontale Ausdehnung (horizontal distance) kommt bei steigender Belastung sogar zum Erliegen und wächst nicht weiter an.
   - Bleibt sehr kompakt und verändert seine Form nur minimal.
   - Geringe Rückstellkraft → sehr flexibel

Für die Schlauchlänge 300 mm wächst die horizontale Ausdehnung des Schlauches bei vertikaler Belastung mit dem Eigengewicht des Schlauches (Schwerkraft) wächst schrittweise an von 25 mm bis 48 mm bei 150 mm (Vertikal Distance down)

Für die Schlauchlänge 600 mm wächst die horizontale Ausdehnung des Schlauches bei vertikaler Belastung mit dem Eigengewicht des Schlauches (Schwerkraft) wächst schrittweise an von 18 mm bis 32 mm bei 125 mm (Vertikal Distance down)

Die horizontale Ausdehnung des Schlauches bei vertikaler Belastung mit dem Eigengewicht des Schlauches (Vertikal Distance down) ist jedoch nicht größer als die (Länge der) angelegte(n) vertikale Belastung (Vertikal Distance down).

Die horizontale Ausdehnung des Schlauches bei vertikaler Belastung mit dem Eigengewicht des Schlauches (Vertikal Distance down) ist als immer annähernd gleichgroß (bis zu +15%) wie die (Länge der) angelegte(n) vertikale Belastung (Vertikal Distance down) oder kleiner.
2. WLM Standard (5)
   - Hat eine sehr hohe horizontale Ausdehnung bei Belastung.
   - Besonders bei 400 mm und 600 mm Länge erreicht der Schlauch Werte von bis zu 295 mm.
   - Hohe Stabilität, aber weniger flexibel.
3. WLM Reuse (6)
   - Mittlere bis hohe horizontale Ausdehnung, insbesondere bei längeren Schlauchsegmenten (600 mm bis 195 mm).
   - Kompromiss zwischen Stabilität und Flexibilität.
4. Plastiflex (8)
   - Zeigt eine mittlere horizontale Ausdehnung, aber die Werte sind inkonsistent (z. B. 400 mm Länge: Sprung von 230 mm auf 88 mm).
   - Weist je nach Belastung unterschiedliche Verformungseigenschaften auf.
5. Cozy Line (7)
   - Vergleichsweise geringe horizontale Ausdehnung (max. 143 mm bei 300 mm Länge).
   - Nicht so flexibel wie andere Modelle.

### Technische Interpretation der Unterschiede

- Schläuche mit hoher horizontaler Ausdehnung (z. B. WLM Standard Ø15) sind weniger flexibel, aber sehr stabil. Sie eignen sich für Anwendungen, bei denen die Formstabilität entscheidend ist.
- Schläuche mit niedriger horizontaler Ausdehnung (z. B. UF Ø15) sind sehr flexibel, passen sich Bewegungen gut an
- Mittlere Werte (z. B. WLM Reuse Ø15, Plastiflex Ø15) bieten einen Kompromiss aus Stabilität und Flexibilität

In den Diagrammen sieht man, dass die horizontale Ausdehnung (Horizontal Distance Out) bei den Schläuchen Cozy Line und Plastiflex ab einer vertikalen Belastung von 150 mm plötzlich stark abnimmt.

### Mögliche Gründe für diesen Einbruch:

Die Reduzierung der horizontalen Auslenkung liegt vor allem daran, dass der Schlauch einen begrenzten Biegeradius aufweist. Der plötzliche Einbruch kommt daher, dass der Schlauch seine maximal messbare Verformung bei 150mm erreicht hat.

Der Einbruch der horizontalen Ausdehnung zeigt, dass Cozy Line und Plastiflex unter zunehmender, vertikaler Last ihre strukturelle Integrität verlieren. In der Praxis bedeutet das, dass sie zwar sehr flexibel sind, aber unter Belastung schnell nachgeben, einknicken oder sich plastisch verformen. Sie sind daher besser für Anwendungen geeignet, bei denen maximale Flexibilität gefragt ist, aber nicht für Anwendungen, bei denen ein formstabiler Schlauch erforderlich ist.

Vorteile des Verhaltens des UF-Schlauches in diesem Bereich:
Der UF Ø15-Schlauch zeigt in den Tabellen und Diagrammen eine sehr geringe Veränderung der horizontalen Ausdehnung, selbst bei steigender vertikaler Belastung.

Der große Vorteil ist, dass der UF-Schlauch bereits bei sehr geringer vertikaler Auslenkung sein maximum an Flexibilität (Biegeverformbarkeit) aufweist. Die anderen Schläuche erreichen diesen Punkt erst bei deutlich stärkerer vertikaler Belastung oder überhaupt nicht aufgrund ihrer zu hohen Steifigkeit

Die gegebene Wertetabelle für 300 mm für UF zeigt eine Funktion, bei der die Werte zunächst ansteigen und sich dann bei 48 stabilisieren.

Die Werte steigen zuerst nicht-linear an und erreichen dann ein Plateau. Eine mögliche Funktion wäre eine asymptotische Funktion wie: $f \left(x\right) = a - b ⋅ {e}^{- c . xf}$

Durch Regression kann die Funktion für 300 mm für UF bestimmt werden beispielsweise als: $f \left(x\right) = 51.40 - 39.60 ⋅ {e}^{- 0.0146 . x}$

Die berechnete Funktion zur Beschreibung der Werte für 600 mm für UF ist beispielsweise: $f \left(x\right) = 33.29 - 25.22 ⋅ {e}^{- 0.0199 . x}$

Diese Funktion zeigt, dass sich die Werte asymptotisch dem Maximalwert von etwa 32 annähern.

Das bedeutet:
1. Hohe Formstabilität
   ∘ Der Schlauch bleibt konstant in seiner Form, auch wenn er belastet wird.
   ∘ Es gibt keinen plötzlichen Einbruch oder Einknicken wie bei Cozy Line oder Plastiflex.
2. Verhindert Luftflussbehinderungen
   ∘ Da sich der Schlauch nicht unkontrolliert verformt oder zusammensackt, bleibt der Luftfluss stabil.
   ∘ Das ist besonders wichtig für Beatmungsschläuche, da eine konstante Luftversorgung gewährleistet sein muss.
3. Erhöhte Langlebigkeit und geringere Materialermüdung
   ∘ UF verliert seine elastischen Eigenschaften nicht so schnell unter Belastung.
   ∘ Das deutet darauf hin, dass das Material entweder eine hohe Rückstellkraft besitzt und eine intelligente Materialkombination verwendet wird.
4. Gute Balance zwischen Flexibilität und Stabilität
   ∘ Während Cozy Line und Plastiflex zu weich und instabil sind, scheint UF einen guten Mittelweg gefunden zu haben.
   ∘ Das ermöglicht eine angenehme Handhabung ohne die Nachteile eines instabilen Schlauchs.

### Mögliche Material- oder Materialkombinationen für diesen Vorteil

1. Silikon mit verstärktem Gewebe
   ∘ Silikon ist flexibel, temperaturbeständig und langlebig.
   ∘ Eine innere Verstärkung durch textile Fasern oder ein dünnes Drahtgeflecht könnte für zusätzliche Stabilität sorgen.
2. Thermoplastische Elastomere (TPE) ggfs. mit Polyurethan (PU)-Beschichtung
   ∘ TPE sorgt für Flexibilität und Elastizität, sodass der Schlauch gut biegbar bleibt.
   ∘ PU sorgt für eine gewisse Steifigkeit und Widerstandsfähigkeit gegen Einknicken.
3. Mehrschichtiger Aufbau mit harter Außenschicht und weicher Innenschicht
   ∘ Äußere Schicht aus Polypropylen (PP) oder Polycarbonat (PC) für Formstabilität.
   ∘ Innere Schicht aus flexiblem Silikon oder Latex für angenehme Biegsamkeit.
   ∘ So bleibt der Schlauch beweglich, aber stabil.
4. Helix zur Verstärkung
   ∘ Ein Schlauch mit einer eingearbeiteten oder aufgesetzten Spirale aus Nylon, Silikon oder einem TPE oder TPE-S kann helfen, die Form zu erhalten.

Der UF-Schlauch weist eine Materialkombination auf, die ihm sowohl Flexibilität als auch Formstabilität verleiht. Eine flexible Membran und eine steife Helix, die beide dauerhaft miteinander verbunden sind, sind ein Aspekt, der zu den Eigenschaften beiträgt.

Die optimale Mischung aus einem elastischen Material (Silikon oder TPE oder TPE-S) für die Membran und einer Verstärkungsschicht, in Form eines Gewebes oder einer Helix oder einer Beschichtung, die die Membran umgibt, ist beispielsweise dafür verantwortlich. Im Falle einer Helix, hat diese versteifende Eigenschaften und ist bevorzugt aus einem anderen Material als die Membran hergestellt. Das macht den Schlauch ideal für Anwendungen, in denen der Schlauch sich gut anpassen muss, aber trotzdem nicht kollabieren darf. Die Materialien lassen sich bevorzugt im Spritzgussverfahren verarbeiten, auch in Mehrkomponenten-Spritzgussanwendungen. Die Materialien sind bevorzugt hautfreundlich und für medizinische Anwendungen geeignet.

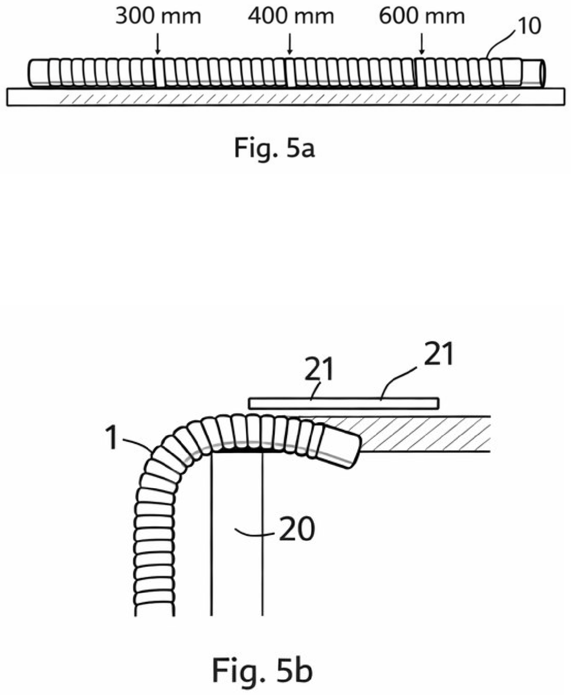

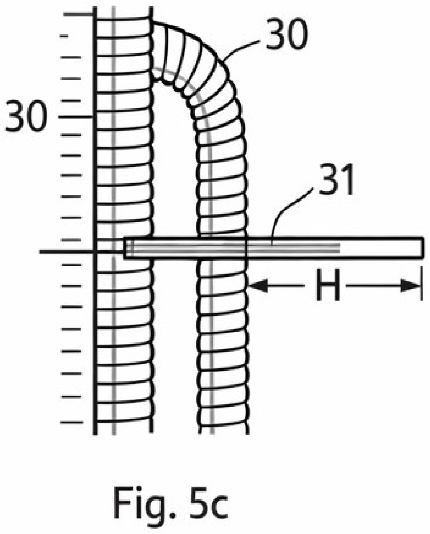

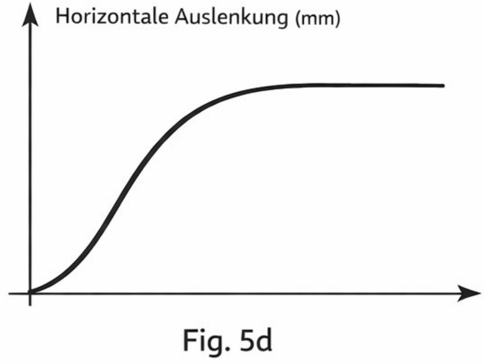

Die Figuren 5 zeigen das Messverfahren für die Drapierbarkeit

Es gibt keine standardisierte Kennzahl für die Drapierbarkeit wie bei der Flexibilität, aber sie kann qualitativ durch Beobachtung oder experimentell durch Biegeprüfungen und Belastungstests bestimmt werden.

Die Drapierbarkeit eines Stoffes kann auf verschiedene Weise getestet werden. Hier sind einige gängige Methoden:
1. Drapiertest: Eine kreisförmige Stoffprobe wird auf eine Platte gelegt und hängt aufgrund der Schwerkraft herab. Die Drapierbarkeit wird durch den Drapierungskoeffizienten gemessen, der das Verhältnis der projizierten Fläche zur ursprünglichen Fläche darstellt.
2. DIN EN ISO 21765: Diese internationale Norm beschreibt eine standardisierte Methode zur Bestimmung der Verformbarkeit von Textilien durch erzwungene Umformung. Sie ermöglicht vergleichbare und reproduzierbare Messwerte zur Drapierbarkeit.

Vorliegend wurde in Anlehnung an die Norm DIN EN ISO 21765 versucht, die Drapierbarkeit zu bestimmten. Jedoch ist die DIN EN ISO 21765 für Textilen anwendbar, die deutlich flexibler sind. Daher wurde eine abgewandelte Messmethode angewendet.

Bei der Prüfung der Drapierbarkeit wirkt nur die Gewichtskraft (F = m x g) als Biegekraft auf den Schlauch. Das sich daraus ergebende Verhältnis aus "Horizontaler Verformung" zu "vertikaler Verformung" gibt dann ein Maß für die Drapierbarkeit an.

Der Schlauch wird mit einer Länge von 300 mm über die Kante einer 90° angeordneten geraden Fläche gehalten, so dass sich der Schlauch aufgrund der Schwerkraft, mit einem gewissen Radius nach unten neigt. Die Drapierbarkeit wird ermittelt indem, auf der vertikalen Höhe von -25 mm, der horizontale Punkt ermittelt wird, an dem sich der Schlauch mit einem geraden Richtmaß überschneidet. Dieses Prozedere wird in vertikalen 25mm Schritten wiederholt. (-50 mm, -75mm, -100mm, bis eine vertikale Länge von -300mm erreicht ist oder bis sich der Schlauch nicht mehr mit dem horizontalen Richtmaß trifft. Die entsprechend ermittelten horizontalen Kreuzungspunkte sind in Abhängigkeit zur vertikalen Richtmaß Position in einem X/Y-Diagramm darzustellen.

Die Drapierbarkeit des Atemschlauchs wird beispielsweise gemessen, indem ein Lineal in einem Abstand von 25mm entlang der vertikalen Messskala geführt wird, bis entweder 300mm erreicht werden oder der Schlauch das Lineal nicht mehr kreuzt. Der Punkt, an dem der Schlauch das Lineal kreuzt, wird als Messstelle notiert. Dieser Vorgang wird bei den 400 und 600 mm Markierungen wiederholt

Das nachfolgend beschriebene Messverfahren dient der quantitativen Ermittlung der Biegeeigenschaften eines flexiblen Beatmungsschlauchs, insbesondere der horizontalen Auslenkung des Schlauchs in Abhängigkeit von einer zunehmenden vertikalen Auslenkung, die ausschließlich durch das Eigengewicht des Schlauchs hervorgerufen wird (Drapierbarkeit).

Figur 5a zeigt die Vorbereitung der Schlauchprobe zur Definition der Prüflängen und Ausgangsgeometrie.
Darstellung:
   - Längsansicht eines geraden Beatmungsschlauchs (1)
   - Markierungen bei 300 mm, 400 mm und 600 mm
   - Darstellung einer Längsmessvorrichtung oder Rinne
Bezugszeichen:
   - (1) Beatmungsschlauch
   - (10) Markierungen der Prüflängen

Ein zu prüfender Beatmungsschlauch wird in einer geraden, unbelasteten Ausgangslage bereitgestellt. Der Schlauch weist in dem zu prüfenden Abschnitt keine Knickstellen, bleibenden Verformungen oder Vorschädigungen auf.

An dem Schlauch werden definierte Prüflängen markiert, beispielsweise:
- 300 mm,
- 400 mm und
- 600 mm,
gemessen jeweils von einem Schlauchende entlang der Längsachse des Schlauchs.

Figur 5b zeigt die Positionierung des Schlauchs an der Haltekante zur Veranschaulichung der Einspannung ohne Klemmen und der rein gravitationsbedingten Belastung.
Darstellung:
   - Schlauch (1) liegt mit einer Markierung auf einer horizontalen Kante (20)
   - Freihängender Schlauchabschnitt unterhalb der Kante
   - Führungselement (21) - beispielsweise ein Rohr - oberhalb der Kante gegen Hochbiegen
Bezugszeichen:
   - (20) Haltekante
   - (21) Führungselement

Figur 5c zeigt die Messung der horizontalen Auslenkung zur Definition der horizontalen Auslenkung bei einer bestimmten vertikalen Auslenkung (Drapierbarkeit).
Darstellung:
   - Vertikale Skala (30) neben dem Schlauch
   - Horizontales Messlineal (31) auf einer definierten vertikalen Höhe
   - Schnittpunkt zwischen Schlauch und horizontalem Lineal
   - Eingezeichneter horizontaler Messabstand (H)
Bezugszeichen:
   - (30) Vertikale Skala
   - (31) Horizontales Messlineal
   - (H) Horizontale Auslenkung

### i)Einspannung und Ausgangslage

Der Schlauch wird mit der jeweiligen Markierung an einer horizontalen Kante einer Haltevorrichtung positioniert, sodass der Schlauchabschnitt ab der Markierung frei nach unten hängt.

Der Schlauch liegt dabei im Bereich der Kante formschlüssig an, ohne eingespannt oder geklemmt zu werden, sodass keine zusätzlichen Haltekräfte außer der Eigenreibung wirken. Oberhalb der Kante wird der Schlauch durch ein Führungselement (21) oder eine Auflage gegen ein ungewolltes Hochbiegen gesichert.

Der frei hängende Schlauchabschnitt wird ausschließlich durch die Schwerkraft belastet und nimmt eine stabile Ruhelage ein.

### ii)Festlegung der vertikalen Auslenkung

Parallel zur hängenden Schlauchlänge ist eine vertikale Skala angeordnet, deren Nullpunkt auf Höhe der Schlauchauflagekante liegt.

Ausgehend von dieser Kante wird die vertikale Auslenkung in diskreten Schritten nach unten gemessen, bevorzugt in 25-mm-Schritten, beginnend bei 25 mm bis zu einer maximalen vertikalen Auslenkung von beispielsweise 300 mm oder bis zu dem Punkt, an dem der Schlauch keine messbare horizontale Auslenkung mehr aufweist.

### iii)Ermittlung der horizontalen Auslenkung

Auf jeder vorgegebenen vertikalen Messhöhe wird ein horizontales Messlineal oder eine horizontale Messebene so positioniert, dass diese:
- senkrecht zur vertikalen Skala ausgerichtet ist und
- sich auf der jeweiligen vertikalen Höhe befindet, ohne den Schlauch zu berühren.

Die horizontale Auslenkung wird als der horizontale Abstand zwischen der vertikalen Referenzebene (durch die Schlauchauflagekante) und dem Schnittpunkt des Schlauchs mit der horizontalen Messebene definiert.

Dieser Wert wird für jede vertikale Auslenkung erfasst und dokumentiert.

### Wiederholung für unterschiedliche Schlauchlängen

Die Schritte i) bis iii) werden für jede zuvor markierte Schlauchlänge (z. B. 300 mm, 400 mm, 600 mm) wiederholt, um das Biegeverhalten des Schlauchs in Abhängigkeit von der wirksamen Schlauchlänge zu erfassen.

### Figur 5d zeigt die Diagrammatische Ergebnisdarstellung

Darstellung:
- Diagramm mit:
   ∘ X-Achse: Vertikale Auslenkung (mm)
   ∘ Y-Achse: Horizontale Auslenkung (mm)
- Nichtlinear ansteigende Kurve mit anschließendem Plateau

Veranschaulichung des charakteristischen Biegeverhaltens des Schlauchs.

Die gemessenen Werte der horizontalen Auslenkung werden in Abhängigkeit von der jeweiligen vertikalen Auslenkung in einem Koordinatensystem dargestellt, wobei:
- die vertikale Auslenkung auf der Abszisse und
- die horizontale Auslenkung auf der Ordinate aufgetragen wird.

Der so entstehende Kurvenverlauf charakterisiert das Drapier- und Biegeverhalten des Schlauchs.

Ein nichtlinearer Anstieg der horizontalen Auslenkung mit anschließendem Übergang in einen Bereich mit im Wesentlichen konstanter horizontaler Auslenkung wird als Hinweis auf ein besonders günstiges Zusammenspiel aus Flexibilität und Formstabilität gewertet.

## Patentansprüche

1. Beatmungsschlauch (1) mit
- einer durchgehenden, elastischen Membran (2), die ein inneres Lumen zur Leitung von Atemgas begrenzt,
- einer die Membran (2) auf der dem Lumen abgewandten Seite spiralförmig umgebenden Helix (3),
wobei
- die Membran (2) aus einem ersten polymeren Material und die Helix (3) aus einem zweiten, hiervon verschiedenen polymeren Material besteht,
- die Helix (3) formschlüssig und/oder stoffschlüssig mit der Membran (2) verbunden ist,
**dadurch gekennzeichnet,**
**dass** die Rückstellkraft des Beatmungsschlauchs höchstens 0,6 N beträgt und dass bei einer Biegeprüfung unter ausschließlicher Einwirkung des Eigengewichts des Schlauches die horizontale Auslenkung des Schlauches mit zunehmender vertikaler Auslenkung zunächst nichtlinear zunimmt und anschließend einen im Wesentlichen konstanten Wert erreicht.

2. Beatmungsschlauch nach Anspruch 1, **dadurch gekennzeichnet, dass** bei der Biegeprüfung die horizontale Auslenkung ab einer vertikalen Auslenkung von höchstens 150 mm bei weiterer Erhöhung der vertikalen Auslenkung eine Änderung von weniger als 10 % aufweist.

3. Beatmungsschlauch nach Anspruch 1 oder 2, **dadurch gekennzeichnet, dass** die Rückstellkraft höchstens 0,4 N, bevorzugt höchstens 0,2 N beträgt.

4. Beatmungsschlauch nach einem der vorhergehenden Ansprüche, dass die Rückstellkraft des Beatmungsschlauchs, gemäß dem in der Beschreibung angegebenen Prüfverfahren gemessen wird.

5. Beatmungsschlauch nach einem der vorhergehenden Ansprüche,
**dadurch gekennzeichnet, dass** die horizontale Auslenkung des Schlauches bei einer vertikalen Auslenkung von 100 mm höchstens 100 mm beträgt.

6. Beatmungsschlauch nach einem der vorhergehenden Ansprüche,
**dadurch gekennzeichnet, dass** sich die Rückstellkraft des Schlauches bei einer Temperatur von 20 °C und bei einer Temperatur von 40 °C um weniger als 20 % unterscheidet.

7. Beatmungsschlauch nach einem der vorhergehenden Ansprüche,
**dadurch gekennzeichnet, dass** die Membran (2) aus einem thermoplastischen Elastomer (TPE), TPE-S oder Silikon besteht.

8. Beatmungsschlauch nach einem der vorhergehenden Ansprüche,
**dadurch gekennzeichnet, dass** die Wanddicke der Membran (2) im Bereich von 0,1 mm bis 0,3 mm liegt.

9. Beatmungsschlauch nach einem der vorhergehenden Ansprüche,
**dadurch gekennzeichnet, dass** die Helix (3) einen Durchmesser von 0,4 mm bis 2,5 mm aufweist.

10. Beatmungsschlauch nach einem der vorhergehenden Ansprüche,
**dadurch gekennzeichnet, dass** die Helix (3) aus TPE, TPE-S oder Silikon hergestellt ist.

11. Beatmungsschlauch nach einem der vorhergehenden Ansprüche,
**dadurch gekennzeichnet, dass** die Helix (3) dem Schlauch eine Knickstabilität verleiht, wobei bei einer Biegung des Schlauches mit einem Radius von 20 mm kein Zusammenfallen des Lumens auftritt.

12. Beatmungsschlauch nach einem der vorhergehenden Ansprüche,
**dadurch gekennzeichnet, dass** die Membran (2) und die Helix (3) im Mehrkomponenten-Spritzgussverfahren oder durch Umspritzen hergestellt sind.

13. Beatmungsschlauch nach einem der vorhergehenden Ansprüche,
**dadurch gekennzeichnet, dass** der Schlauch für die Anwendung in einer CPAP-, BiPAP- oder invasiven Beatmungstherapie ausgelegt ist, wobei der Schlauch mit einer durchgehenden Membran (2) ausgekleidet ist, die ein inneres Lumen begrenzt, wobei das Lumen zur Leitung von Atemgas ausgebildet ist und eine Helix (3) die Membran (2) auf der dem Lumen abgewandten Seite spiralförmig umgibt, wobei der der Innendurchmesser 15 - 22 mm ist und die Rückstellkraft des Schlauches bevorzugt unter 0,6 N ist und wobei der Schlauch an einem Ende eine Muffe aufweist, die dem Anschluss an ein Beatmungsgerät dient und an dem anderen Ende einen Adapter aufweist, der der Ankopplung einer Beatmungsmaske dient.
